# EUROPÄISCHE PATENTANMELDUNG

(11) **EP 0 935 953 A2**
(43) Veröffentlichungstag der Anmeldung: **18.08.1999**
(21) Anmeldenummer: 99102554.5
(22) Anmeldetag: 10.02.1999
(51) Int. Cl.: A61F 2/16

(54) **Intraocularlinse**

(30) Priorität: 12.02.1998 DE 19805780
(71) Anmelder: Anschütz, Till Dr., 76571 Gaggenau (DE)
(72) Erfinder: Anschütz, Till Dr., 76571 Gaggenau (DE)
(74) Vertreter: Nöth, Heinz, Dipl.-Phys.

(57) **Zusammenfassung**

Eine Intraocularlinse mit einem optischen Linsenteil, der im Auge in der Vorderkammer vor der Iris implantierbar ist, und Haptikschlaufen 3, 4, welche im implantierten Zustand durch Schnitte in der Iris hindurchgeführt sind und in der Hinterkammer des Auges, insbesondere im Bereich des Sulcus ciliaris abgestützt sind.

## Beschreibung

Die Erfindung betrifft eine Intraocularlinse nach dem Oberbegriff des Patentanspruches 1.

Eine derartige aus der WO 97/27 825 bekannte Intraocularlinse besitzt Haptikschlaufen, welche an die Peripherie des Linsenkörpers angeformt sind. Die aus der US 5 628 796 bekannte Linse besitzt Stützfüßchen, welche durch Öffnungen an der Peripherie der Iris ragen. Die aus der US 3 986 214 bekannte Linse weist ebenfalls radial vom Linsenkörper abstehende Stützfüßchen auf.

Die aus der EP 0 061 282 B1 bekannte Intraocularlinse besitzt einen optischen Linsenteil, der im Auge in der Vorderkammer vor der von der Iris gebildeten Pupille implantiert wird. Zur Abstützung dienen federnd ausgebildete Haptikschlaufen, die an drei Stellen im Kammerwinkel der Vorderkammer des Auges mit Fußplatten abgestützt werden.

Aufgabe der Erfindung ist es, eine Vorderkammerlinse zu schaffen, welche eine einfach ausgebildete Haptik aufweist, mit welcher eine lagestabile Abstützung des optischen Linsenteils im Auge erreicht wird.

Diese Aufgabe wird erfindungsgemäß durch die kennzeichnenden Merkmale des Patentanspruches 1 gelöst.

Bei der Erfindung wird die Haptik von Fäden bzw. offenen Schlaufen gebildet, die bezüglich der optischen Linsenachse einen radial innenliegenden Faden- bzw. Schlaufenteil und einen radial außenliegenden Faden- bzw. Schlaufenteil aufweisen. Der radial innenliegende Schlaufenteil ist mit dem optischen Linsenteil verbunden, und der radial außenliegende Schlaufenteil dient zur Abstützung in der Hinterkammer des Auges, insbesondere am Sulcus ciliaris hinter der Iris. Hierzu sind die radial außenliegenden Schlaufenteile mit gegenüber der Ebene des optischen Linsenteils schräg verlaufenden Schlaufenteilen verbunden. Die radial außenliegenden Schlaufenteile werden bei der Implantation durch Schnitte im Bereich des Außenrandes der Iris hindurchgeführt, so daß sie am Sulcus ciliaris hinter der Iris in der Hinterkammer des Auges abstützbar sind. Die radial außenliegenden Schlaufenteile liegen in einer Ebene, die etwa parallel zu der Ebene liegt, in welcher die Verbindungsstelle der innenliegenden Schlaufenteile mit dem optischen Linsenteil vorgesehen sind. Die radial innenliegenden Schlaufenteile können im Linsenkörper des optischen Linsenteils eingegossen sein. Es ist jedoch auch möglich, die radial innenliegenden Faden- bzw. Schlaufenteile am Umfang des optischen Linsenteils anzuformen. Die Verbindungsstellen liegen bezüglich der optischen Achse des optischen Linsenteils an diametralen Stellen. In bevorzugter Weise können die Verbindungsstellen auf Kreisabschnitten liegen, insbesondere wenn die offenen Haptikschlaufen bzw. -fäden im Linsenkörper eingebettet bzw. eingegossen sind. Der Linsenkörper kann aus faltbaren Material, beispielsweise aus einem Silikonpolymer oder Hydrogelpolymer bestehen. Die offenen Haptikschlaufen bzw. -fäden bestehen aus einem härteren Material beispielsweise Polymethylmetacrylat (PMMA). Zum vereinfachten Hindurchfädeln der Haptikschlaufen durch die in die Iris gelegten Schnitte können die freien Enden der Haptikschlaufen mit keulenförmigen Verdickungen versehen sein.

In vorteilhafter Weise wird durch die Ertindung eine Vorderkammerlinse insbesondere fake Vorderkammerlinse geschaffen, die in der Hinterkammer des Auges bevorzugt am Sulcus ciliaris abgestützt ist.

Anhand der Figuren wird an einem Ausführungsbeispiel die Erfindung noch näher erläutert. Es zeigt:
- Fig. 1:: eine Draufsicht auf ein Ausführungsbeispiel der Erfindung;
- Fig. 2:: das Ausführungsbeispiel im implantierten Zustand in schnittbildlicher Darstellung; und
- Fig. 3:: das Ausführungsbeispiel im implantierten Zustand in Draufsicht.

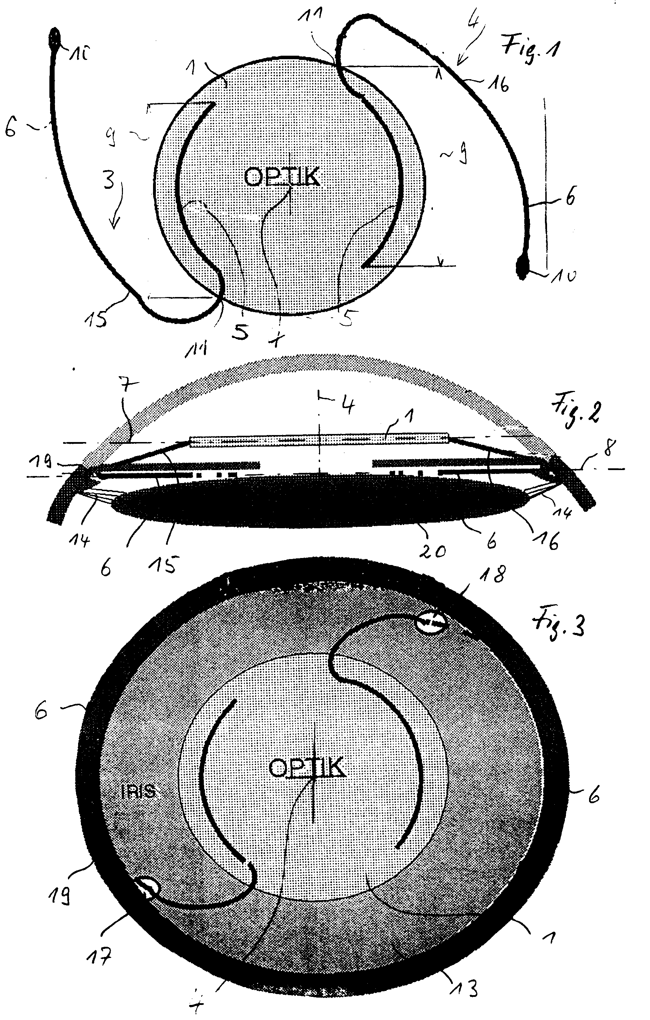

Das Ausführungsbeispiel besitzt einen optischen Linsenteil 1. Im Linsenkörper des optischen Linsenteils 1 sind offene Haptikschlaufen (Fäden) 3 und 4, beispielsweise durch Eingießen, eingebettet. Die fadenförmigen Haptikschlaufen haben eine etwa U- bzw. V-Form mit gebogenen, insbsondere etwa kreisbogenförmig ausgebildeten Schlaufenteilen im Endbereich an den jeweiligen Schenkel. Die Haptikschlaufen 3, 4 besitzen radial außenliegende Schlaufenteile 6 und radial innenliegende Schlaufenteile 5. Die innenliegenden Schlaufenteile 5 sind entlang einer gekrümmten insbesondere kreisförmig gekrümmten Verbindungsstelle 9 mit dem optischen Linsenteil 1 verbunden. Beim dargestellten Ausführungsbeispiel sind die innenliegenden Fadenteile 5 mit dem optischen Linsenteil beispielsweise während dessen Polymerisation eingegossen und eingebettet worden. Bezüglich der optischen Achse 4 des Linsenteils 1 liegen die Verbindungsstellen 9 bzw. die radial innenliegenden Schlaufenteile 5 diametral zueinander. Auch Austrittstellen 11 der Haptikschlaufen aus dem Linsenkörper des optischen Linsenteiles 1 liegen bezüglich der optischen Achse diametral an der Peripherie des Linsenteiles 1. Die radial innenliegenden Schlaufenteile 5 liegen in einer zur optischen Achse 4 senkrechten Verbindungsebene 7, in welcher die auf den Kreisabschnitten vorgesehenen Verbindungsstellen 9 der innenliegenden Fadenteile 5 mit dem optischen Linsenteil 1 liegen. Die radial außenliegenden Fadenteile 6 liegen in einer Stützebene 78 in welcher sie im Sulcus ciliaris 12 hinter der Iris 13 und vor den Zonulafasern 14 im Auge abgestützt sind. Die beiden Ebenen 7 und 8 liegen parallel und in Richtung der optischen Achse 4 versetzt zueinander. Zwischen den radial innenliegenden Fadenteilen 5 und den radial außenliegenden Fadenteilen 6 erstrecken sich Schlaufenteile 15, 16, welche im Winkel zu den beiden Ebenen 7 und 8 verlaufen. Durch den schrägen Verlauf der Fadenteile 15, 16 wird der Abstand des optischen Linsenteils 1 von der Iris bzw. vom Sulcus ciliaris und der natürlichen Augenlinse 20 bestimmt, wie es aus der Fig. 2 ersichtlich ist. Wie insbesondere aus der Fig. 3 zu ersehen ist, liegen die radial außenliegenden Schlaufenteile 6 innerhalb eines Kreisabschnittes im Sulcus ciliaris an. Die sich abstützenden Kreisabschnitte der radial außenliegenden Schlaufenteile 6 liegen bevorzugt etwa konzentrisch zu den Kreisabschnitten der Verbindungsstellen 9, an denen die radial innenliegenden Schlaufenteile 5 mit dem Linsenteil 1 verbunden sind, wobei der gemeinsame Mittelpunkt der entlang den Kreisabschnitten verlaufenden Schlaufenteile 5, 6 von der optischen Achse 4 des Linsenteiles 1 gebildet wird. Auf diese Weise wird mit einfachen Mitteln bei der Abstützung des optischen Linsenteiles 1 in der Vorderkammer eine ausbalancierte Haltekraft auf den optischen Linsenteil 1 ausgeübt, so daß dieser in der Vorderkammer positionsgerecht und stabil vor der von der Iris 13 gebildeten Pupille und der natürlichen Augenlinse 20 angeordnet ist. Diese stabile Positionierung des optischen Linsenteils 1 ergibt sich insbesondere auch dadurch, daß die Kreisabschnitte der innen- und außenliegenden Schlaufenteile 5, 6 diametral zueinander liegen. Dies gilt auch für korrespondierende Stellen an den schräg verlaufenden Schlaufen- bzw. Fadenteilen 15, 16, die die innen- und außenliegenden Schlaufenteilen 5, 6 miteinander verbinden. Der Durchmesser des optischen Linsenteils 1 beträgt etwa 6,0 bis 6,5 mm, und der Abstand der radial am weitesten außen liegenden Stellen der außen liegenden Schlaufenteile 6 voneinander beträgt etwa 12,5 bis 13,5 mm. Der Abstand (ca. 3 mm) der beiden Ebenen 7 und 8 in axialer Richtung ist so bemessen, daß eine stabile Positionierung des optischen Linsenteils 1 vor der Pupille erreicht wird. Diese stabile Positionierung wird noch durch die zusätzliche Abstützung der Haptikschlaufen, insbesondere im Bereich der Schlaufenteile 15, 16 an der Iris unterstützt.

Die Intraocularlinse kann als aphake Linse nach einer Katarektoperation oder als phake Linse vor der natürlichen Augenlinse implantiert werden.

## Patentansprüche

1. Intraocularlinse mit einem optischen Linsenteil, der im Auge in der Vorderkammer vor der Iris implantierbar ist, und federnd ausgebildeten Haptikschlaufen, welche jeweils radial innenliegende mit dem optischen Teil verbundene Schlaufenteile und radial außenliegende an intraocularen Abstützstellen abstützbare Schlaufenteile aufweist, wobei die radial außenliegenden Schlaufenteile gegenüber den radial innenliegenden Schlaufenteilen in Richtung der optischen Achse des optischen Linsenteils zur Hinterkammer des Auges versetzt sind, die radial außenliegenden Schlaufenteile durch Schnitte im Bereich des Außenrandes der Iris hindurchführbar sind und hinter der Iris, insbesondere am Sulcus ciliaris abstützbar sind; radial innenliegenden und die die radial außenliegenden Schlaufenteile in entlang der optischen Achse des optischen Linsenteils versetzten Ebenen liegen und zwischen den radial innen- und außenliegenden Schlaufenteilen im Winkel zu den beiden Ebenen verlaufende Schlaufenteile vorgesehen sind,
dadurch **gekennzeichnet,**
daß die Haptikschlaufen (3, 4) eine etwa U- oder V-Form, deren jeweilige Schenkel von den innen- und außenliegenden Schlaufenzeilen (5, 6) gebildet sind, aufweisen und daß die radial innenliegenden Schlaufenteile (5) im Linsenkörper des optischen Linsenteils (1) eingebettet sind, und die radial außen liegenden Schlaufenteile (6) entlang konzentrischen Kreisabschnitten gebogen sind.

2. Intraocularlinse nach einem der Anspruch 1 dadurch gekennzeichnet, daß zueinander korrespondierende Schlaufenteile der Haptikschlaufen (3, 4) bezüglich der optischen Achse diametral zueinander liegen.

3. Intraocularlinse nach Anspruch 1 oder 2, dadurch gekennzeichnet, daß an den freien Enden der außen liegenden Schlaufenteile (6) Verdickungen (10) vorgesehen sind.

4. Intraocularlinse nach einem der Ansprüche 1 bis 3, dadurch gekennzeichnet, daß die radial innenliegenden Schlaufenteile (5) im Linsenkörper des optischen Linsenteils (1) eingebettet sind.

5. Intraocularlinse nach einem der Ansprüche 1 bis 4, dadurch gekennzeichnet, daß die Verbindungsstellen (9) auf Kreisabschnitten liegen.

6. Intraocularlinse nach Anspruch 5, dadurch gekennzeichnet, daß die Kreisabschnitte der Verbindungsstellen (9) um die optische Achse (4) angeordnet sind.

7. Intraocularlinse nach einem der Ansprüche 1 bis 3, dadurch gekennzeichnet, daß die radial innen liegenden Schlaufenteile (5) an den Umfang des optischen Linsenteils (1) angeformt sind.

8. Intraocularlinse nach einem der Ansprüche 1 bis 7, dadurch gekennzeichnet, daß zueinander korrespondierende Schlaufenteile der Haptikschlaufen (3,4) bezüglich der optischen Achse diametral zueinander liegen.

9. Intraocularlinse nach einem der Ansprüche 1 bis 8, dadurch gekennzeichnet, daß die radial außen liegenden Schlaufenteile (6) gebogen sind und insbesondere entlang Kreisabschnitten gebogen sind.

10. Intraocularlinse nach einem der Ansprüche 1 bis 9, dadurch gekennzeichnet, daß an den freien Enden der außen liegenden Schlaufenteilen (6) Verdickungen (10) vorgesehen sind.
